(19) Europäisches Patentamt
European Patent Office
Office européen des brevets

(11) EP 1 714 965 A1

(12) EUROPEAN PATENT APPLICATION
published in accordance with Art. 158(3) EPC

(43) Date of publication:
25.10.2006 Bulletin 2006/43

(21) Application number: 05709799.0

(22) Date of filing: 07.02.2005

(51) Int Cl.:
*C07D 453/02* (2006.01) *A61K 31/439* (2006.01)
*A61P 13/02* (2006.01) *A61P 27/08* (2006.01)
*A61P 1/12* (2006.01)

(86) International application number:
PCT/JP2005/001747

(87) International publication number:
WO 2005/075474 (18.08.2005 Gazette 2005/33)

(84) Designated Contracting States:
AT BE BG CH CY CZ DE DK EE ES FI FR GB GR HU IE IS IT LI LT LU MC NL PL PT RO SE SI SK TR

(30) Priority: 09.02.2004 US 542472 P

(71) Applicant: Astellas Pharma Inc.
Tokyo 103-8411 (JP)

(72) Inventors:
- ISHII, Yusuke,
  c/o Astellas Pharma Inc.
  Tokyo 1038411 (JP)
- TAKAOKA, Kouji,
  c/o Astellas Pharma Inc.
  Tokyo 1038411 (JP)
- INAKOSHI, Masatoshi,
  c/o Astellas Pharma Inc.
  Tokyo 1038411 (JP)
- NAKAGAWA, Shuichi,
  c/o Astellas Pharma Inc.
  Tokyo 1038 (JP)
- NAGATA, Koji
  c/o Astellas Pharma Inc.
  Chuo-ku, Tokyo 103-8411 (JP)
- YORIMOTO, Naoki,
  c/o Astellas Pharma Inc.
  Tokyo 1038411 (JP)
- TAKEUCHI, Makoto,
  c/o Astellas Pharma Inc.
  Tokyo 1038411 (JP)
- YONETOKU, Yasuhiro,
  c/o Astellas Pharma Inc.
  Tokyo 1038411 (JP)

(74) Representative: HOFFMANN EITLE
Patent- und Rechtsanwälte
Arabellastrasse 4
81925 München (DE)

# (54) COMPOSITION CONTAINING SOLIFENACIN SUCCINATE

(57) A solifenacin succinate-containing composition with less impurities which can be used as a bulk for pharmaceutical is provided.

The solifenacin succinate-containing compostiion of the present invention has a reduced content of especially its optical isomers in comparison with the known compositions containing an acid addition salt of solifenacin, so that it can be used for production of a therapeutic agent containing solifenacin succinate. In addition, the above-described solifenacin succinate-containing composition can be easily produced in accordance with the production method of the present invention.



Printed by Jouve, 75001 PARIS (FR)

## Description

Technical Field

The present invention relates to a composition containing solifenacin succinate at a low content of impurities and for use as a drug, particularly as a muscarine $M_3$ receptor antagonist, more specifically as a bulk for therapeutic agents of for example urological diseases such as pollakiuria and incontinence of urine due to hyperactive bladder, as well as a method for producing the same.

Background Art

It is required that a bulk as an active ingredient which is a raw materials for pharmaceutical products should be at high purity. In the Case Tokyo High Court Heisei 9 (Gyo ke) No. 302 determined on February 17, 2000, for example, the decision remarks that "when diagnostic agents and therapeutic agents contain even a trace amount of impurities above the acceptable limits, obviously, a possibility of some adverse influence on diagnosis and therapeutic treatment cannot be denied, and it is a technical common sense in the art to which the subject invention belongs." That is, it is a technical common sense that it is important for pharmaceutical products to obtain a bulk at high purity with as low as possible for impurities

Accordingly, in order to obtain a highly pure bulk containing impurities as less as possible in producing bulks, an active ingredient in a pharmaceutical product produced by chemical synthesis is repeatedly subjected to purification processes such as purification steps by various chromatographic means, distillation step, and/or crystallization step and subsequent recrystallization step to be carried out once or several times, so that the active ingredient has an increased purity for use as a bulk.

Concerning impurities in pharmaceutical preparations, further, guidelines based on the agreement at the International Harmonization Association for the Japan/US/EU Pharmaceutical Products Regulations have been established (see for example "The Guideline about Impurities in Bulk among New Active Ingredient-Containing Pharmaceutical Products" attached to "The Revision of the Guideline about Impurities in Bulk among New Active Ingredient-Contaiaing Pharmaceutical Products", which is issued as No. 1216001 by the Pharmaceutical Council (Iyakushin) as dated December 16, 2002).

Particularly, therapeutic agents of pollakiuria and incontinence of urine due to hyperactive bladder are used not as agents for curing hyperactive bladder itself but as therapeutic agents for suppressing the symptoms thereof, namely pollakiuria and incontinence of urine. Accordingly, it is expected that these therapeutic agents are administered for a long period of time. Therefore, it is readily expected that a particularly high purity is required for these therapeutic agents, unlike pharmaceutical agents in forms of potion drugs to be administered temporarily.

On the other band, the chemical name of solifenacin is (1*S*, 3'*R*)-quinuclidin-3'-yl 1-phenyl-1,2,3,4-tetrahydroisoquinoline-2-carboxylate and has the following chemical formula.

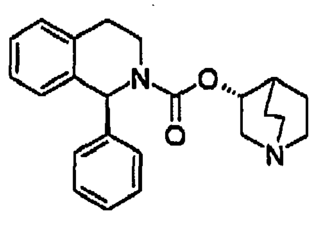

Solifenacin or salts thereof are compounds known as muscarine $M_3$ receptor antagonists (patent reference 1, non-patent reference 1, non-patent reference 2, non-patent reference 3). It is under clinical trials in order to use as a therapeutic agent for pollakiuria and incontinence of urine due to hyperactive bladder. Additionally, reports tell about its efficacy on, for example, interstitial cystitis (patent reference 2), tension relaxation of ciliary muscle (patent reference 3), and irritable bowel syndrome (non-patent reference 4).

Because solifenacin or salts thereof have two asymmetric carbons, it is not easy to produce a bulk containing solifenacin or a salt thereof at a high purity by eliminating optical isomers thereof. On the other hand, it is important for the use as a bulk.

Among the technical references described above, the patent reference 1 merely describes methods for producing solifenacin, solifenacin hydrochloride, and solifenacin oxalate in detail. However, none of the references include any suggestion or indication about a detailed method or purification method about a pharmaceutical composition containing solifenacin succinate. It is needless to say that none of them includes any suggestion or description about the composition thereof.

Additionally, there is a report about the investigation of the crystallization conditions in order to improve the fluidity of solifenacin succinate, which greatly influence on the formulation and the handling at plants for the bulk, by controlling the particle size of solifenacin succinate. However, the report only tells about the investigation of the crystallization conditions in order to improve the fluidity. No suggestion or description is included therein about the composition of a pharmaceutical composition containing solifenacin succinate and/or the control of impurities (non-patent reference 5).

[Patent reference 1] Specification of European Patent No. 801067

[Patent reference 2] Pamphlet of International Publication WO 2003/6019

[Patent reference 3] Laid-open publication of JP-A-2002-104968

[Non-patent reference 1] Current Opinion in Central & Peripheral Nervous System Investigational Drugs, 2000, Vol.2, No.3, p.321-325

[Non-patent reference 2] Drugs of the Future, 1999, Vol.24, No.8, p.871-874

[Non-patent reference 3] Naunyn-Schmiedeberg's Archives of Pharmacology, 2002, Vol.366, No.2, p.97-103

[Non-patent reference 4] Japanese Journal of Pharmacology, 2001, Vol.86, No.3, p.281-288

[Non-patent reference 5] The Abstract of Lectures at the Memorial Symposium for the Foundation and Establishment of Japan Process Chemistry Association (held on July 4 to 5, 2002), p.85-86.

Disclosure of the Invention

Problems to Be Solved by the Invention

The present inventors found that a solifenacin-contaming composition produced by chemical synthesis contained compound A through compound E shown by the following formulas as main impurities and that these compounds reduced the purity of the solifenacin-containing composition.

Compound A

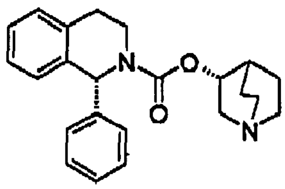

Compound B

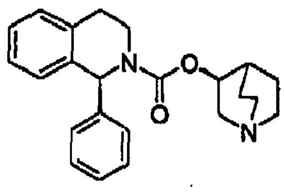

Compound C

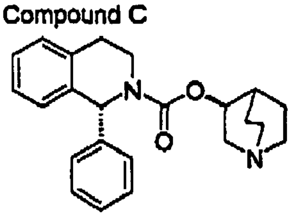

Compound D

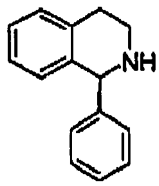

Compound E

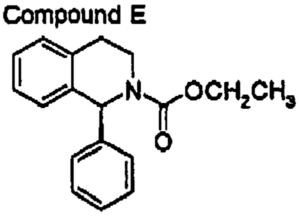

The inventors found that it was very important to remove these compounds or acid addition salts thereof as impurities as much as possible for using, as a bulk, the solifenacin-containing compound or a composition containing an acid addition salt of solifenacin as prepared by modifying it into the acid addition salt. Since the compound A, the compound B and the compound C in particular among the compounds are optical isomers of solifenacin, however, the removal thereof was very difficult compared with the other compounds.

Concerning solifenacin hydrochloride and solifenacin oxalate as solifenacin or salts thereof, of which the production methods were known in detail, the inventors found that by crystallizing in an appropriate solvent the individual solifenacin acid-addition salts resulting from the reaction of solitfenacin with corresponding acids and the subsequent distillation and removal of the reaction solvents as shown in Reference Example 2 and Reference Example 4 mentioned below, the resulting compositions contained the compound A and the compound B at 0.85% or more and 0.50% or more, respectively. That is, in order to use the solifenacin hydrochloride-containing composition and the solifenacin oxalate-containing composition as a bulk, the inventors found that these compositions essentially required further plural crystallization steps such as recrystallization.

In order to use solifenacin produced by chemical synthesis as a bulk, development of a method for producing a composition containing solifenacin or an acid addition salt thereof and containing less impurities in a simple manner is strongly desired.

Means for Solving the Problems

The inventors made investigations on a method for producing a composition containing solifenacin or an acid addition salt thereof with less impurities, as well as a purification method thereof. Consequently, the inventors found that a composition containing a solifenacin acid-addition salt with far less impurities as never obtained by using hydrochloric acid or oxalic acid was obtained by reacting a solifenacin-containing composition produced by chemical synthesis with succinic acid and then crystallizing the composition in the form of the succinate salt. Thus, the invention has been achieved.

According to the invention, a solifenacin succinate-containing composition and particularly a solifenacin succinate-containing pharmaceutical composition are provided, which comprise the compound represented by the formula (I)

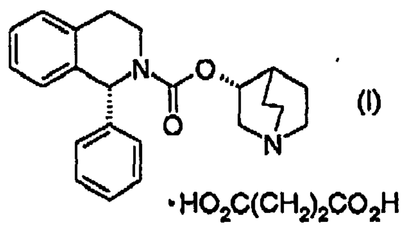

(I)

at 0.8% or less, more preferably at 0.1% or less to solifenacin succinate.

The invention further provides a method for producing such solifenacin-containing composition comprising adding succinic acid in a solvent for salt formation to a composition containing solifenacin, a salt thereof, a solvate thereof, or a solvate of a salt thereof to prepare a solifenacin succinate-containing solution and depositing the solifenacin succinate-containing composition in the solution; a method comprising adding succinic acid in a solvent for salt formation to a crude reaction composition containing solifenacin, a salt thereof, a solvate thereof, or a solvate of a salt thereof to prepare a solifenacin succinate-containing solution and depositing the solifenacin succinate-containing composition in the solution; a method comprising adding succinic acid in a solvent for salt formation to a crude reaction composition containing solifenaciua, a salt thereof, a solvate thereof, or a solvate of a salt thereof, which is preliminarily prepared by a reaction between a compound represented by the formula (III)

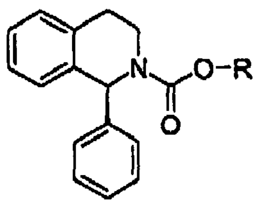

(III)

[in the formula, R represents a lower alkyl] and (*R*)-quinuclidin-3-ol, to prepare a solifenacin succinate-containing solution and depositing the solifenacin succinate-containing composition in the solution; or a method comprising adding succinic acid in a solvent for salt formation to a crude reaction composition containing solifenacin, a salt thereof, a solvate thereof, or a solvate of a salt thereof, which is preliminarily prepared by a reaction between a compound represented by the formula (III) and (*R*)-quinuclidin-3-ol in the presence of an alkali metal lower alkoxide, to prepare a solifenacin succinate-containing solution and depositing the solifenacin succinate-containing composition in the solution.

The invention furthermore provides a solifenacin succinate-containing composition and particularly a solifenacin succinate-containing pharmaceutical composition comprising the compound represented by the above formula (I) at 0.8% or less, more preferably at 0.1% or less to solifenacin succinate, which are produced by the methods described above.

In accordance with the invention, a solifenacin succinate-containing composition and particularly a solifenacin succinate-containing pharmaceutical composition comprising the compound represented by the formula (II)

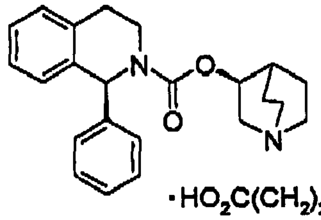

(II)

$\cdot HO_2C(CH_2)_2CO_2H$

at 0.4% or less, more preferably at 0.2% or less to solifenacin succinate.

As methods for producing them, there are provided a method for producing such composition including adding succinic acid in a solvent for salt formation to a composition containing solifenacin, a salt thereof, a solvate thereof or a solvate of a salt thereof to prepare a solifenacin succinate-containing solution and depositing the solifenacin succinate-containing composition in the solution; a method comprising adding succinic acid in a solvent for salt formation to a crude reaction composition containing solifenacin, a salt thereof, a solvate thereof, or a solvate of a salt thereof to prepare a solifenacin succinate-containing solution and depositing the solifenacin succinate-containing composition in the solution; a method comprising adding succinic acid in a solvent for salt formation to a crude reaction composition containing solifenacin, a salt thereof, a solvate thereof, or a solvate of a salt thereof, which is prepared by a reaction between a compound represented by the formula (III)

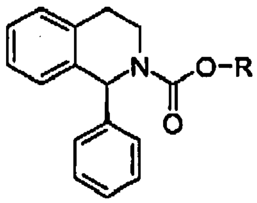

(III)

[in the formula, R represents a lower alkyl] and (*R*)-quinuclidin-3-ol, to prepare a solifenacin succinate-containing solution and depositing the solifenacin succinate-containing composition in the solution; or a method comprising adding succinic acid in a solvent for salt formation to a crude reaction composition containing solifenacin, a salt thereof, a solvate thereof, or a solvate of a salt thereof, which is prepared by a reaction between a compound represented by the above formula (III) and (*R*)-quinuclidin-3-ol in the presence of an alkali metal lower alkoxide, to prepare a solifenacin succinate-containing solution and depositing the solifenacin succinate-containing composition in the solution.

In accordance with the invention, a solifenacin succinate-containing composition and particularly a solifenacin succinate-containing pharmaceutical composition as produced by the methods described above are provided, which contain the compound represented by the above formula (II) at 0.4% or less, more preferably at 0.2% or less to solifenacin succinate.

Effect of the Invention

The patent reference 1 above describes the specific production method of a solifenacin hydrochloride-containing composition and a solifenacin oxalate-containing composition. Therefore, a solifenacin succinate-containing composition can readily be produced according to it As shown in the Examples, however, the solifenacin hydrochloride-containing composition and the solifenacin oxalate-containing composition contain compound A and compound B at 0.85% or more and 0.50% or more to solifenacin, respectively, even after salt formation and crystallization steps.

The solifenacin succinate-containing composition in accordance with the present invention contains compound A and compound B as less as 0.1% or less and 0.2% or less to solifenacin, respectively, which can be produced in a simple manner by cooling a reaction solution after a salt formation step and collecting the deposited crystal by filtration.

For using the solifenacin hydrochloride-containing composition and the solifenacin oxalate-containing composition as pharmaceutical products, these compositions should be subjected to additional recrystallization steps plural times. However, the solifenacin succinate-containing composition can be obtained as a composition for use as a pharmaceutical product in such a simple manner by cooling a reaction solution after a salt formation step and collecting the

deposited crystal by filtration.

In other words, quite unexpectedly, a composition containing an acid addition salt of solifenacin can be produced in a simple manner by using succinic acid, where the compound A and compound B as the impurities are contained at 0.8% or less, preferably 0.1% or less and 0.4% or less, preferably 0.2% or less, respectively. The resulting composition itself is never obvious to a person skilled in the art.

Brief Description of Drawings

Fig. 1: A chart of impurities of compound A, compound B and compound C in the solifenacin-containing ethyl acetate solution obtained in Reference Example 1, as measured by HPLC. A peak at a retention time of about 32.5 minutes shows solifenacin; peaks at retention times of about 17.9 minutes, about 21.5 minutes and about 19.0 minutes show compound A, compound B and compound C, respectively.

Fig. 2: A chart of impurities of compound A, compound B and compound C in the solifenacio hydrochloride-containing composition obtained in Reference Example 2, as measured by HPLC. A peak at a retention time of about 32.3 minutes shows solifenacin; peaks at retention times of about 17.4 minutes, about 21.1 minutes and about 18.8 minutes show compound A, compound B and compound C, respectively.

Fig. 3: A chart of impurities of compound A, compound B and compound C in the solifenacin hydrochloride-containing composition obtained in Reference Example 3, as measured by HPLC. A peak at a retention time of about 32.1 minutes shows solifenacin; a peak at a retention time of about 17.4 minutes shows compound A.

Fig. 4: A chart of impurities of compound A, compound B and compound C in the solifenacin oxalate-containing composition obtained in Reference Example 4, as measured by IIPLC. A peak at a retention time of about 32.4 minutes shows solifenacin; and peaks at retention times of about 17.4 minutes and about 21.1 minutes show compound A and compound B, respectively.

Fig. 5: A chart of impurities of compound A, compound B and compound C in the solifenacin suecinate-containing composition obtained in Example 2, as measured by HPLC. A peak at a retention time of about 32.5 minutes shows solifenacin; peaks at retention times of about 18.0 minutes and about 21.5 minutes show compound A and compound B, respectively.

Fig. 6: A chart of impurities of compound A, compound B and compound C in the solifenacin succinate-containing composition obtained in Example 3, as measured by HPLC. A peak at a retention time of about 35.8 minutes shows solifenacin; and a peak at a retention time of about 23.5 minutes shows compound B.

Best Mode for Carrying out the Invention

In accordance with the invention, the term "lower alkyl", means a linear or branched $C_{1-6}$ alkyl and specifically includes, for example, methyl, ethyl, propyl, butyl, pentyl or hexyl, or structural isomers thereof such as isopropyl or tert-butyl. Preferably, it is methyl, ethyl, propyl and isopropyl and particularly preferable one is ethyl.

The term "alkali metal lower alkoxide" means a salt between a linear or branched $C_{1-6}$ alcohol and an alkali metal, where the alkali metal includes, for example, lithium, sodium and potassium, preferably sodium and potassium and more preferably sodium. The "alkali metal alkoxide" specifically includes, for example, sodium methoxide, sodium ethoxide, sodium propoxide, sodium isopropoxide, sodium butoxide, potassium methoxide, potassium ethoxide, aud potassium tert-butoxide. Preferably, it is sodium methoxide, sodium ethoxide and potassium tert-butoxide, more preferably, sodium ethoxide.

The term "solvent for salt formation" means any solvent which is generally used in reactions for modifying basic substances such as solifenacin into acid addition salts thereof and includes, for example, organic solvents, water, or mixtures thereof. More specifically, the solvent includes, for example, alcohols such as methanol, ethanol, 1-propanol, 2-propanol, 1-butanol, 2-butanol, and tert-butanol; ethers such as diethyl ether, diitsopropyl ether, tetrahydrofuran, dioxane, and dimethoxyethane; ketones such as acetone and methyl ethyl ketone; esters such as ethyl acetate, n-propyl acetate, n-butyl acetate, methyl propionate, and ethyl propionate; aprotic polar solvents such as N,N-dimethylformamide, N,N-dimethylacetamide, N-methylpyrrolidone, and dimethylsulfoxide; acetonitrile; halogenated hydrocarbons such as dichloromethane, chloroform, and 1,2-dichloroethane; aromatic hydrocarbons such as benzene, toluene and xylene; saturated hydrocarbons such as hexane and heptane; water; or mixture solvents of any types of solvents selected from them. Preferably, it is one or more solvents selected from the group consisting of ethers, esters and alcohols, or mixture solvents thereof; more preferably, a mixture solvent of alcohols and esters; particularly preferably a mixture solvent of ethanol and ethyl acetate.

The term "a salt thereof' in the "solifenacin, a salt thereof, a solvate thereof, or a solvate of a salt thereof" means a salt of solifenacin with a pharmaceutically acceptable acid and specifically includes acid addition salts of solifenacin

with inorganic acids such as hydrochloric acid and sulfuric acid; or organic acids such as acetic acid, oxalic acid and malonic acid. Herein, the "solifenacin, a salt thereof, a solvate thereof, or a solvate of a salt thereof is preferably solifenacin.

The term "crude reaction composition" means a solifenacin-containing composition obtained by post-treatments such as procedures for solution partition and extraction procedure after the completion of the reaction at the step of producing solifenacin, and does not include the solifenacin-containing composition obtained by carrying out purification procedures for the solifenacin-containing composition, including purification procedures by various chromatographic means, distillation procedure, crystallization procedure and/or recrystallization procedure. The reaction as the step of producing solifenacin which is used for producing such crude reaction composition specifically includes, for example, a step of reacting a compound represented by the formula (III)

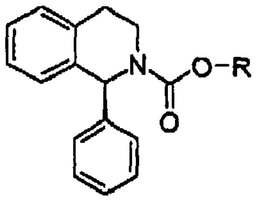

(III)

[in the formula, R represents a lower alkyl] with (*R*)-quinuclidin-3-ol. The composition of the formula (III) and (*R*)-quinuclidin-3-ol may be compounds at 100% optical purities and may also be compounds containing optical isomers thereof at 5% or less, preferably 3% or less and more preferably 1% or less.

The content ratios described in the instant specification all represent the area ratios by HPLC analysis, when the area of solifenacin succinate is defined as 100%. The content ratios are measured under the following conditions for the HPLC analysis the similar conditions.

Furthermore, the present invention also encompasses compositions including so-called labeled solifenacin succinate, which is a compound produced by substituting a part or the whole of atoms constituting solifenacin succinate with radioisotopes.

The composition of the present invention can be produced by the following production method or a variation thereof.

Succinic acid is added to and dissolved in a reaction solution at the final step of producing solifenacin from, for example, the condensation reaction between a compound represented by the above formula (III) and (*R*)-quinuclidin-3-ol (preferably, the reaction is carried out using a solvent for salt formation), or a solution of a solvent for salt formation as obtained from post-treatments such as extraction, rinsing and/or solvent removal after the final step of producing solifenacin, or a solution prepared by adding to these solutions a solvent for salt formation, which may be a solvent for salt formation of the same type and/or a different type.

Then, succinic acid can be used at an amount 0.5 to 2.0-fold,preferably 0.7 to 1.2-fold, more preferably 0.8 to 1.0-fold the theoretical amount calculated from the final step of producing solifenacin. Additionally, succinic acid may be heated and dissolved during the addition and dissolution of succinic acid. Preferable examples of the solvent for salt formation include ethers, esters and alcohols., or may be mixture solvents of one or more solvents selected from the group consisting of these solvents.

By cooling a solution containing solifenacin succinate in a solvent for salt formation as obtained above, the solifenacin succinate-containing composition in accordance with the present invention can be deposited. The deposited composition can be filtered and recovered by the general method, washed using an appropriate solvent and dried, so that the solifenacin succinate-containing composition in accordance with the present invention can be obtained.

Depending on the scale of the step, it is preferable that the cooling rate is not high. As the rinsing solvent, any solvent with such a solubility of solifenacin succinate as is not so large may be used. Preferably, the solvent may be ethers, esters, alcohols, or mixture solvents of one or more solvents selected from the group consisting of these solvents. Drying may also be done with heating, under reduced pressure, or with heating under reduced pressure.

## Examples

The present invention is now described specifically with the following Examples but the present invention is not limited to the Examples. Methods for producing the raw material compound used in the Examples and methods for producing a solifenacin hydrochloride-containing composition and a solifenacin oxalate-containing composition as Comparative Examples are shown in Reference Examples.

Herein, the compositions of impurities in the individual compositions obtained in the following Reference Examples or the Examples were measured as follows.

(Methods for assaying impurities in individual compositions)

1. Method for assaying compound A, compound B and compound C

0.25 g of a composition obtained in the following Reference Examples or Examples was dissolved in a mixed solution of hexane/2-propanol (1:1) to a final total volume of 100 mL to give a test solution as a sample solution. To 1 mL of the sample solution was further added the mixed solution of hexane/2-propanol (1:1) to a final total volume of 100 mL to give a standard solution. 10 μl each of the sample solution and the standard solution was tested by liquid chromatography under the following conditions, to measure the area of each peak of the individual solutions by the automatic integral method. By the following formula, the amount of impurities was calculated.

$$\text{Content ratio (\%) of individual impurities} = \text{ATi/AS}$$

[in the formula, ATi represents the peak area of individual impurities in the sample solution, while AS represents the peak area of solifenacin in the standard solution.]

<Test conditions>

Detector: ultraviolet absorption photometer (wavelength for measurement: 220 nm)
Column: CHIRAL PAK AD-H (250 mm x 4.6 mm ID, manufactured by Daicel Chemical)
Column temperature: 20°C
Mobile phase: hexane/2-propanol/diethylamine mixed solution (800:200:1)
Flow rate: adjusted so that the retention time of solifenacin becomes about 35 minutes (about 1 mL/minute)

2. Method for assaying compound D

0.05 g of a composition obtained in the following Reference Examples or Examples was dissolved in a solution (solution P), which was preliminarily prepared by adding phosphoric acid to a solution of 8.7 g of dipotassium hydrogen phosphate dissolved in water to 1000 mL to adjust the resulting solution to pH 6.0 and then adding 300 mL of acetonitrile to 700 mL of the resulting pH-adjusted solution. Then, the resulting solution was adjusted to a final total volume of 100 mL to give a sample solution. To 1 mL of the sample solution was added the solution P, to a final total volume of 100 mL to give a standard solution. 10 μl each of the sample solution and the standard solution was tested by liquid chromatography under the following conditions, to measure the area of each peak of the individual solutions by the automatic integral method. By the following equation, the amount of impurities was calculated.

$$\text{Content ratio (\%) of individual impurities} = \text{ADTi/ADS}$$

[in the equation, ADTi represents the peak area of individual impurities in the sample solution, while ADS represents the peak area of solifenacin in the standard solution.]

<Test conditions>

Detector: ultraviolet absorption photometer (wavelength for measurement: 210 nm)
Column: Develosil ODS-UG-5 (150 mm x 4.6 mm ID, manufactured by Nomura Chemical) or an equivalent one
Column temperature: 40°C
Mobile phase: Solution P
Flow rate: adjusted so that the retention time of solifenacin becomes about 20 minutes (about 1 mL/minute)

3. Method for assaying compound E

0.05 g of a composition obtained in the following Reference Examples or Examples was dissolved in the solution P to a final total volume of 100 mL to give a sample solution. To 1 mL of the sample solution was added the solution P, to a final total volume of 100 mL, to give a standard solution. 10 μl each of the sample solution and the standard solution was tested by liquid chromatography under the following conditions, to measure the area of each peak of the individual solutions by the automatic integral method. By the following equation, the amount of impurities was calculated.

$$\text{Content ratio (\%) of individual impurities} = \text{AETi/AES}$$

[in the equation, AETi represents the peak area of individual impurities in the sample solution, while AES represents the peak area of solifenacin in the standard solution.]

<Test conditions>

Detector: ultraviolet absorption photometer (wavelength for measurement: 210 nm)
Column: Develosil ODS-UG-5 (150 mm x 4.6 mm ID, manufactured by Nomura Chemical) or an equivalent one
Column temperature: 40°C
Mobile phase: a solution prepared by adding phosphoric acid to a solution of 8.7 g of dipotassium hydrogen phosphate dissolved in water to 1000 mL, adding phosphoric acid to the resulting solution to adjust the resulting solution to pH 6.0 and then adding 500 mL of acetonitrile to 500 mL of the resulting pH-adjusted solution.
Flow rate: adjusted so that the retention time of solifenacin becomes about 3 minutes (about 1 mL/minute)

Because such basic solvents or phosphate buffer is selected as the mobile phase, all detections are for the basic substances after the addition salt is eliminated.

Reference Example 1

Production of solifenacin

A mixture of 360 liters of water and 83.2 kg of potassium carbonate was added to a mixture of 120 kg of (*S*)-1-phenyl-1,2,3,4-tetrahydroisoquinoline and 600 liters of toluene. The resulting mixture was cooled to 10°C, to which 65.3 kg of ethyl chloroformate was subsequently dropwise added, followed by agitation at 25°C for 2 hours. After the aqueous layer was separated, the organic layer was washed with 360 liters of water, After 290 liters of the solvents were distilled off under reduced pressure, 1320 liters of toluene and 81 liters of *N,N*-dimethylformamide were further added, to which 87.5 kg of (*R*)-quinuclidin-3-ol and 7.8 kg of sodium ethoxide were added at room temperature. While distilling off the solvents, the mixture was heated for 8 hours. 480 liters of toluene and 400 liters of water were added to the reaction solution, which is then cooled to room temperature. The aqueous layer was separated. The organic layer was washed with 400 liters of water. The organic layer was further extracted in 77.4 kg of conc. hydrochloric acid and 440 liters of water. Then, a mixture of 126.8 kg of potassium carbonate and 320 liters of water was added to the resulting aqueous layer, followed by extraction with 810 liters of ethyl acetate. After the organic layer was washed with 160 liters of water, 160 liters of ethanol and 240 liters of ethyl acetate were added. 820 liters of the solvent were distilled off from the solution at atmospheric pressure to obtain 527.8 kg of an ethyl acetate solution containing (1*S*, 3'*R*)-quinuclidin-3'-yl 1-phenyl-1,2,3,4-tetrahydroisoquinoline-2-carboxylate (referred to as "solifenacin" hereinafter).

In Table 1, the composition of impurities in the solution is shown as the content ratio when solifenacin is defined as 100%, while Fig. 1 shows the data about the measured compositions of impurities of compound A, compound B and compound C.

Reference Example 2

Production of solifenacin hydrochloride-containing composition

100 mL of the ethyl acetate solution containing solifenacin obtained in Reference Example 1 was concentrated under reduced pressure to obtain an oily matter of 33.9 g. 140 mL of ethanol and 10 mL of 4M hydrochloric acid-ethyl

acetate solution were added to 13.53 g of the oily matter, from which the solvents were distilled off under reduced pressure. 56 mL of acetonitrile and 150 mL of ethyl ether were added to the resulting residue, for crystallization at room temperature, to obtain 8.637 g of a solifenacin hydrochloride-containing composition.

In Table 1, the composition of impurities in the solifenacin hydrochloride-containing composition is shown as the content ratio when solifenacin is defined as 100%, while Fig. 2 shows the data about the measured compositions of impurities of compound A, compound B and compound C.

Reference Example 3

Pwification of solifenacin hydrochloride-containing composition produced in Reference Example 2

50 mL of acetonitrile was added to 5.00 g of the solifenacin hydrochloride-containing composition obtained in Reference Example 2, for dissolution with heating. 78 mL of ethyl ether was added to the solution, and cooled to 25°C. The deposited crystal was collected by filtration to obtain 4.025 g of a solifenacin hydrochloride-containing composition in colorless crystal.

In Table 1, the composition of impurities in the solifenacin hydrochloride-containing composition is shown as the content ratio when solifenacin is defined as 100%, while Fig. 3 shows the data about the measured compositions of impurities of compound A, compound B and compound C.

Reference Example 4

Production of solifenacin oxalate-containing composition

100 mL of the ethyl acetate solution containing solifenacin as obtained in Reference Example 1 was concentrated under reduced pressure to obtain 33.9 g of an oily matter. 100 mL of ethanol and 2.73 g of oxalic acid were added to 11.00 g of the oily matter, from which the solvents were distilled off under reduced pressure. 50 mL of isopropanol and 80 mL of isopropyl ether were added to the resulting oily matter, for dissolution with heating. The solution was cooled to 25°C, and the deposited crystal was collected by filtration to give 8.720g of a solifenacin oxalate-containing composition in colorless crystal.

In Table 1, the composition of impurities in the solifenacin oxalate-containing composition is shown as the content ratio when solifenacin is defined as 100%, while Fig. 4 shows the data about the measured compositions of impurities of compound A, compound B and compound C.

Example 1

Production of seed crystal of solifenacin succinate

60 liters of water and then 23.8 kg of potassium carbonate were added to a mixture of 30.0 kg of (*S*)-1-phenyl-1,2,3,4-tetrahydroisoquinoline and 300 liters of toluene. The resulting mixture was cooled to 10°C, to which 18.7 kg of ethyl chloroformate was subsequently dropwise added, followed by agitation for one hour. After the aqueous layer was separated, the organic layer was washed with 150 liters of water. The organic layer was further washed with 150 liters of water, from which the solvents were distilled off under reduced pressure.

360 liters of toluene and 40 liters of *N,N*-dimethylformamide were added to the resulting residue, to which 21.6 kg of (*R*)-quinuclidin-3-ol and 2.89 kg of sodium ethoxide were added at room temperature. While distilling off the solvents, the mixture was heated for 8 hours. 200 liters of water was added to the reaction solution, which was then cooled to room temperature. The aqueous layer was separated. The organic layer was washed with 200 liters of water. The organic layer was further extracted in 47.6 kg of conc. hydrochloric acid and 360 liters of water. Then, a mixture of 72.5 kg of potassium carbonate and 400 liters of water was added to the resulting aqueous layer, followed by extraction with 400 liters of ethyl acetate. After the organic layer was washed with 100 liters of water, the solvents therein were distilled off. To the resulting residue were added 450 liters of ethyl acetate, 90 liters of ethanol and 14.6 kg of succinic acid, for dissolution with heating. The solution was then cooled to 0°C, and the deposited crystal was collected by filtration, which was then washed with 80 liters of ethyl acetate and dried under reduced pressure, to obtain 46.40 kg of solifenacin succinate.

Example 2

Production of solifenacin succinate-containing composition (1)

To 261.0 kg of the ethyl acetate solution containing solifenacin obtained in Reference Example 1 were added 140 liters of ethanol, 120 liter of ethyl acetate and 31.1 kg of succinic acid, for dissolution with heating. 12 liters of ethanol and 28 liters of ethyl acetate were added to the resulting solution, which was then cooled to 50°C. 9.11 g of solifenacin succinate produced in the same manner as in Example 1 was added. The resulting mixture was cooled to 0°C, and the deposited crystal was collected by filtration. The crystal was washed with 190 liters of ethyl acetate, and dried under reduced pressure to obtain 87.82 kg of a solifenacin succinate-containing composition.

In Table 1, the composition of impurities in the solifenacin succinate-containing composition is shown as the content ratio when solifenacin is defined as 100%, while Fig. 5 shows the data about the measured compositions of impurities of compound A, compound B and compound C.

Example 3

Production of solifenacin succinate-containing composition (2)

To 180 mL of the ethyl acetate solution containing solifenacin as obtained in Reference Example 1 were added 91 mL of ethanol, 78 mL of ethyl acetate and 20.9 g of succinic acid, for dissolution with heating. 8 mL of ethanol and 18 mL of ethyl acetate were added to the resulting solution, which was then cooled to 50°C. 0.02 g of solifenacin succinate produced in the same manner as in Example 1 was added. The resulting mixture was cooled to 30°C, and then again heated to 50°C, and retained at 50°C for 2 hours. Then, the mixture was cooled to 0°C over 5 hours. The deposited crystal was collected by filtration. The resulting crystal was washed with 100 mL of ethyl acetate, and dried under reduced pressure, to obtain 59.135 g of a solifenacin succinate-containing composition.

In Table 1, the composition of impurities in the solifenacin succinate-containing composition is shown as the content ratio when solifenacin is defined as 100%, while Fig. 6 shows the data about the measured compositions of impurities of compound A, compound B and compound C.

(Table 1)

| | Reference Example 1 | Reference Example 2 | Reference Example 3 | Reference Example 4 | Example 2 | Example 3 |
|---|---|---|---|---|---|---|
| Compound A | 4.51% | 1.70% | 0.46% | 0.85% | 0.05% | ND |
| Compound B | 2.33% | 0.50% | ND | 0.67% | 0.17% | 0.07% |
| Compound C | 0.14% | ND | ND | ND | ND | ND |
| Compound D | 0.32% | 0.06% | 0.03% | 0.06% | ND | ND |
| Compound E | 1.07% | 0.16% | ND | 0.09% | ND | ND |

In the table, "ND" means that it is lower than the detection limit. The NDs of compound A, compound B and compound C are about 0.05% or less, while the NDs of compound D and compound E are about 0.01% or less. The compounds A through E in the table are compounds with the following structures.

Compound A

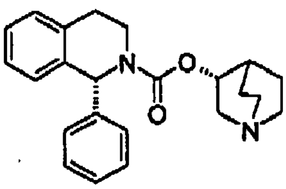

Compound B

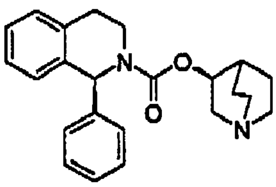

Compound C

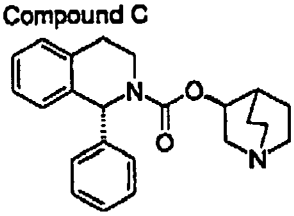

Compound D

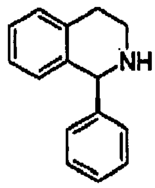

Compound E

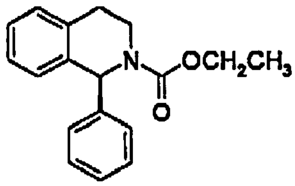

In producing a composition containing an acid addition salt of solifenacin with hydrochloric acid as in Reference Example 2 and in producing a composition containing an acid addition salt of solifenacin with oxalic acid as in Reference Example 4, a purification effect from the individual compounds A through E was observed. However, the compositions contained compound A at 0.85% or more to solifenacin and compound B at 0.5% or more to solifenacin.

In producing a composition containing an acid addition salt of solifenacin with succinic acid as in Example 2 and Example 3, a purification effect from individual compounds A through E was observed. A highly excellent purification effect for compound A and compound B as never observed in the Reference Examples was also obtained.

The solifenacin hydrochloride-containing composition after the recrystallization procedure as shown in Reference Example 3 contained compound A at 0.46% to solifenacin although the recrystallization step was additionally done thereafter.

The aforementioned results apparently indicate that only the solifenacin succinate-containing composition among the compositions containing acid addition salts of solifenacin can be produced as a composition containing highly pure solifenacin or salt thereof in a simple manner.

Industrial Applicability

The solifenacin succinate-containing compostiion of the present invention has a reduced impurity content in comparison with the known compositions containing an acid addition salt of solifenacin, so that it can be used for production of a therapeutic agent containing solifenacin succinate.

In addition, the above-described solifenacin succinate-containing composition can be easily produced in accordance with the production method of the present invention.

## Claims

1. A solifenacin succinate-containing composition, comprising a compound represented by the formula (I)

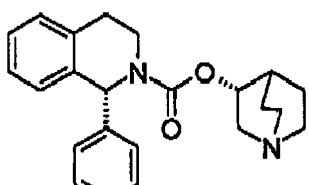

(I)

$\cdot HO_2C(CH_2)_2CO_2H$

at 0.8% or less to solifenacin succinate.

2. A solifenacin succinate-containing composition according to claim 1, comprising a compound represented by the formula (I) according to claim 1 at 0.1% or less to solifenacin succinate.

3. A composition according to any one of claims 1 and 2, which is a pharmaceutical composition.

4. A method for producing a composition according to any one of claims 1, 2 and 3, comprising adding succinic acid in a solvent for salt formation to a composition containing solifenacin, a salt thereof, a solvate thereof or a solvate of a salt thereof to prepare a solifenacin succinate-containing solution, and depositing a solifenacin succinate-containing composition in the solution.

5. A method for producing a composition according to any one of claims 1, 2 and 3, comprising adding succinic acid in a solvent for salt formation to a crude reaction composition containing solifenacin, a salt thereof, a solvate thereof or a solvate of a salt thereof to prepare a solifenacin succinate-containing solution, and depositing a solifenacin succinate-containing composition in the solution.

6. A method according to claim 5, wherein the crude reaction composition is a crude reaction composition produced by a reaction between a compound represented by the formula (III)

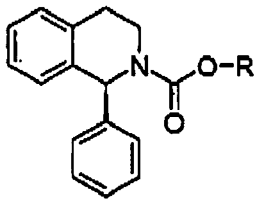

(III)

(in the formula, R represents a lower alkyl) and (*R*)-quinuclidin-3-ol.

7. A method according to claim 6, wherein the crude reaction composition is a crude reaction composition produced by a reaction between a compound represented by the formula (III) according to claim 6 and (*R*)-quinuclidin-3-ol in the presence of an alkali metal lower alkoxide.

8. A method according to any one of claims 4, 5, 6 and 7, wheren the solvent for salt formation is one or more solvents selected from the group consisting of ethers, esters and alcohols or a mixture solvent thereof.

9. A composition according to any one of claims 1, 2 and 3 produced by a method according to any one of claims 4, 5, 6, 7 and 8.

10. A solifenacin succinate-containing composition, comprising a compound represented by the formula (II)

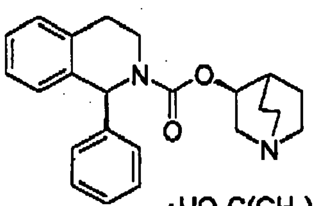

(II)

$\cdot HO_2C(CH_2)_2CO_2H$

at 0.4% or less to solifenacin succinate.

11. A solifenacin succinate-containing composition according to claim 10, comprising a compound represented by the formula (II) according to claim 10 at 0.2% or less to solifenacin succinate.

12. A composition according to any one of claims 10 and 11, which is a pharmaceutical composition.

13. A method for producing a composition according to any one of claims 10, 11 and 12, comprising adding succinic acid in a solvent for salt formation to a composition containing solifenacin, a salt thereof, a solvate thereof or a solvate of a salt thereof to prepare a solifenacin succinate-containing solution, and depositing a solifenacin succinate-containing composition in the solution.

14. A method for producing a composition according to any one of claims 10,11 and 12, comprising adding succinic acid in a solvent for salt formation to a crude reaction composition containing solifenacin, a salt thereof, a solvate

thereof or a solvate of a salt thereof to prepare a solifenacin succinate-containing solution, and depositing a solifenacin succiraate-containing composition in the solution.

**15.** A method according to claim 14, wherein the crude reaction composition is a crude reaction composition produced by a reaction between a compound represented by the formula (III)

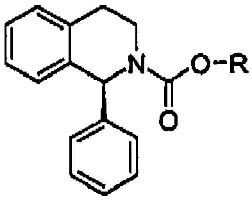

(III)

(in the formula, R represents a lower alkyl) and (*R*)-quinuclidin-3-ol.

**16.** A method according to claim 15, wherein the crude reaction composition is a crude reaction composition produced by a reaction between a compound represented by the formula (III) according to claim 15 and (*R*)-quinuelidin-3-ol in the presence of an alkali metal lower alkoxide.

**17.** A method according to any one of claims 13, 14, 15 and 16, wherein the solvent for salt formation is one or more solvents selected from the group consisting of ethers, esters and alcohols or a mixture solvent thereof.

**18.** A composition according to any one of claims 10,11 and 12 produced by a method according to any one of claims 13, 14, 15, 16 and 17.

FIG. 1

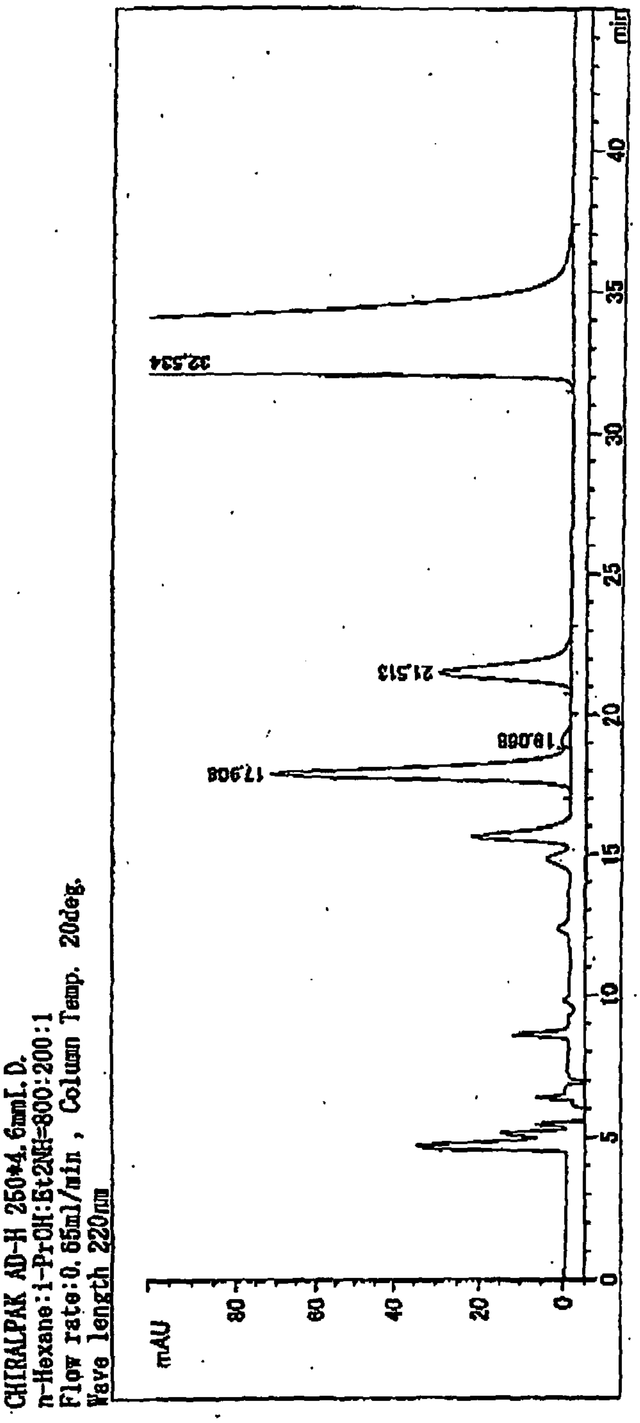

CHIRALPAK AD-H 250*4.6mmI.D.
n-Hexane:i-PrOH:Et2NH=800:200:1
Flow rate:0.65ml/min , Column Temp. 20deg.
Wave length 220nm
mAU
80
60
40
20
0
0
5
10
15
20
25
30
35
40
min
17.908
18.088
21.513
32.534

FIG. 2

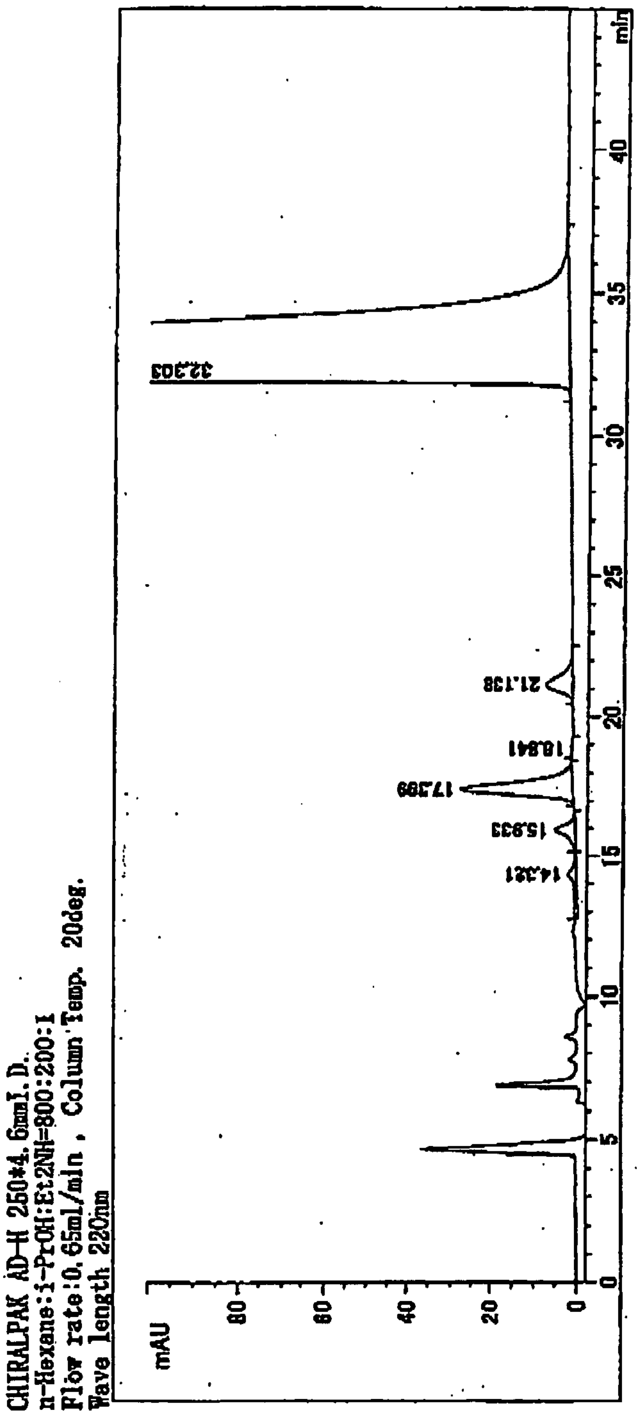
CHIRALPAK AD-H 250*4.6mmI.D.
n-Hexane:i-PrOH:Et2NH=800:200:1
Flow rate:0.65ml/min , Column Temp. 20deg.
Wave length 220nm
mAU
80
60
40
20
0
0
5
10
15
20
25
30
35
40
min
14.321
15.933
17.399
18.841
21.138
32.303

FIG. 3

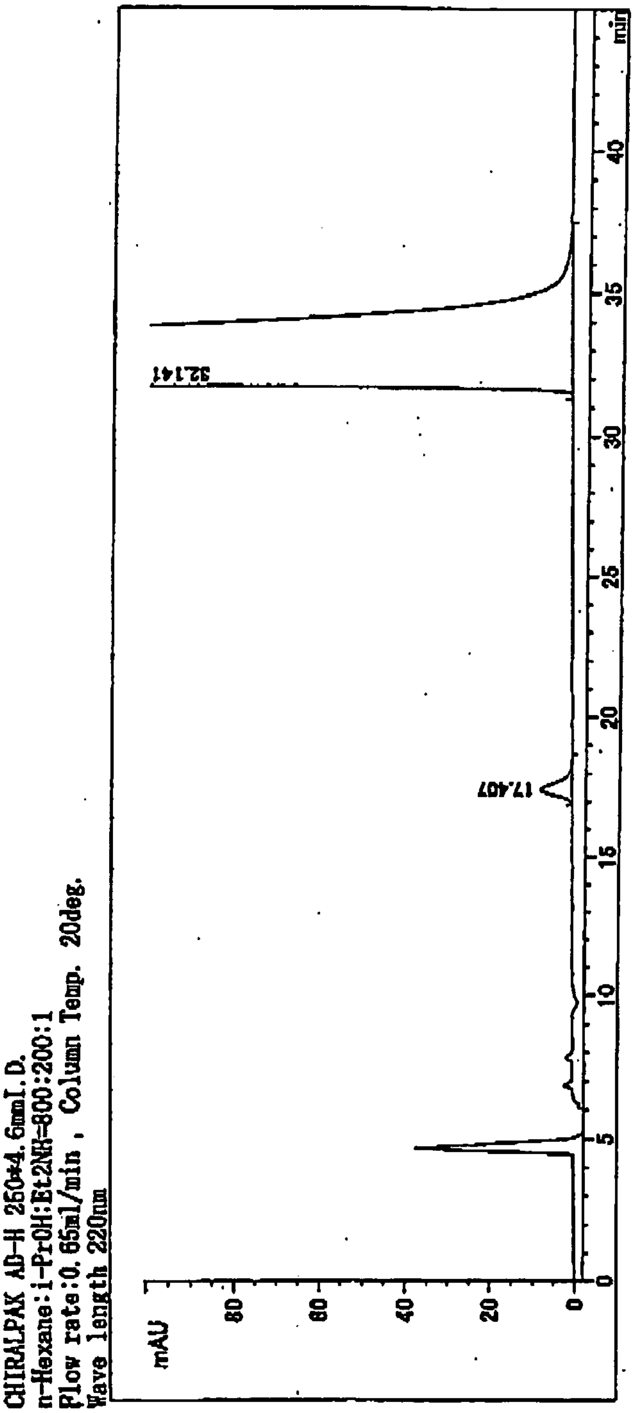
CHIRALPAK AD-H 250*4.6mmI.D.
n-Hexane:i-PrOH:Et2NH=800:200:1
Flow rate:0.65ml/min , Column Temp. 20deg.
Wave length 220nm
mAU
80
60
40
20
0
0
5
10
15
17.407
20
25
30
32.141
35
40
min

FIG. 4

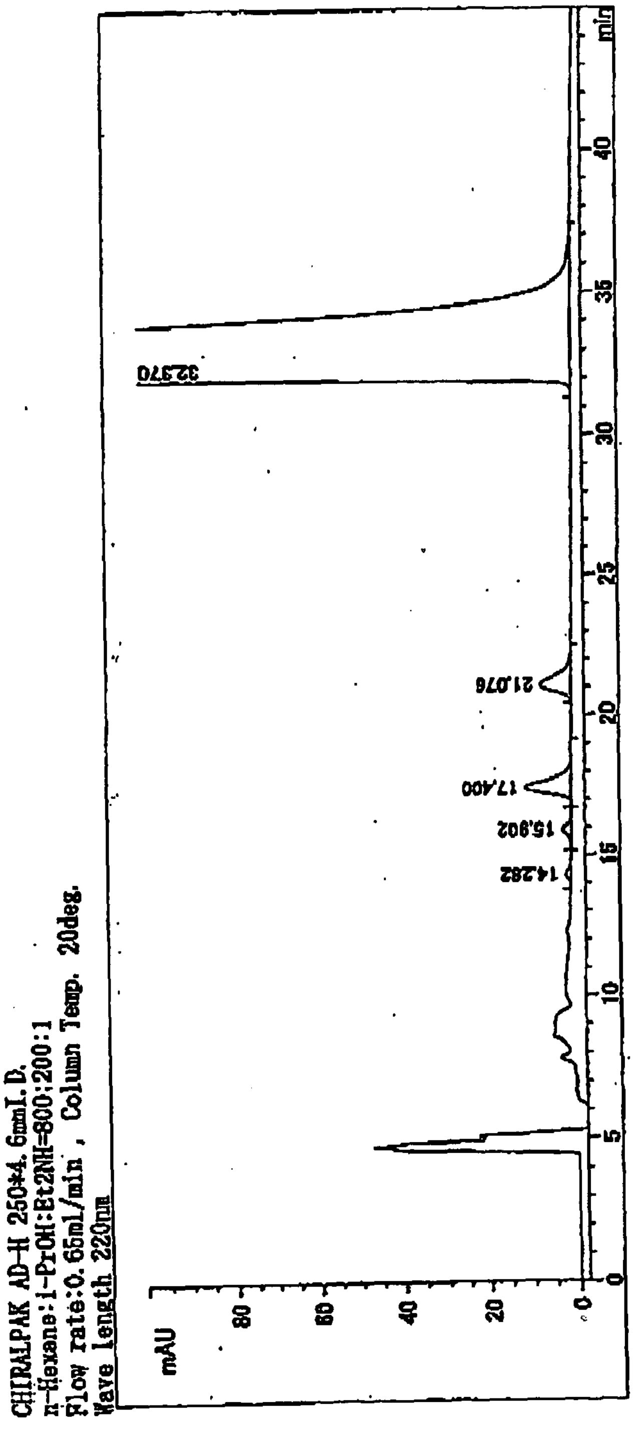
CHIRALPAK AD-H 250*4.6mmI.D.
n-Hexane:1-PrOH:Et2NH=800:200:1
Flow rate:0.65ml/min , Column Temp. 20deg.
Wave length 220nm
mAU
80
60
40
20
0
0
5
10
15
20
25
30
35
40
min
14.282
15.802
17.400
21.076
32.370

FIG. 5

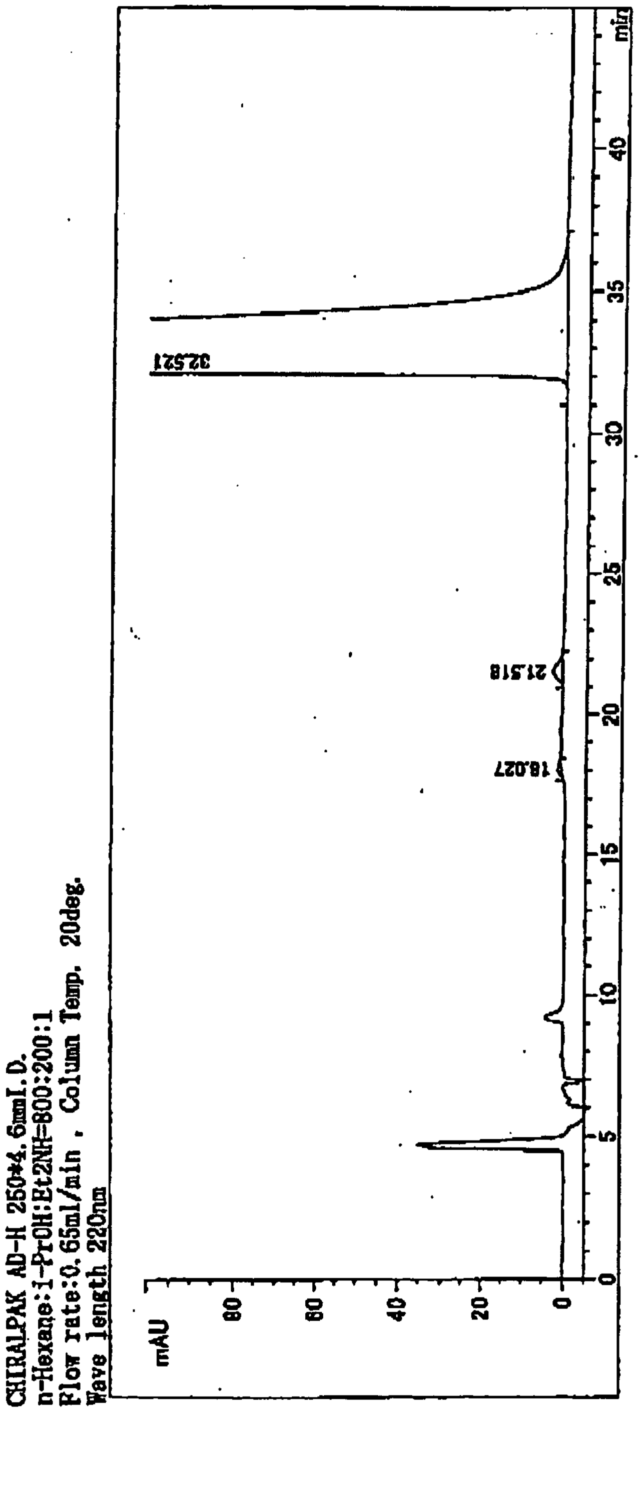
CHIRALPAK AD-H 250*4.6mmI.D.
n-Hexane:1-PrOH:Et2NH=800:200:1
Flow rate:0.65ml/min , Column Temp. 20deg.
Wave length 220nm
mAU
min
32.521
21.518
18.027

FIG. 6

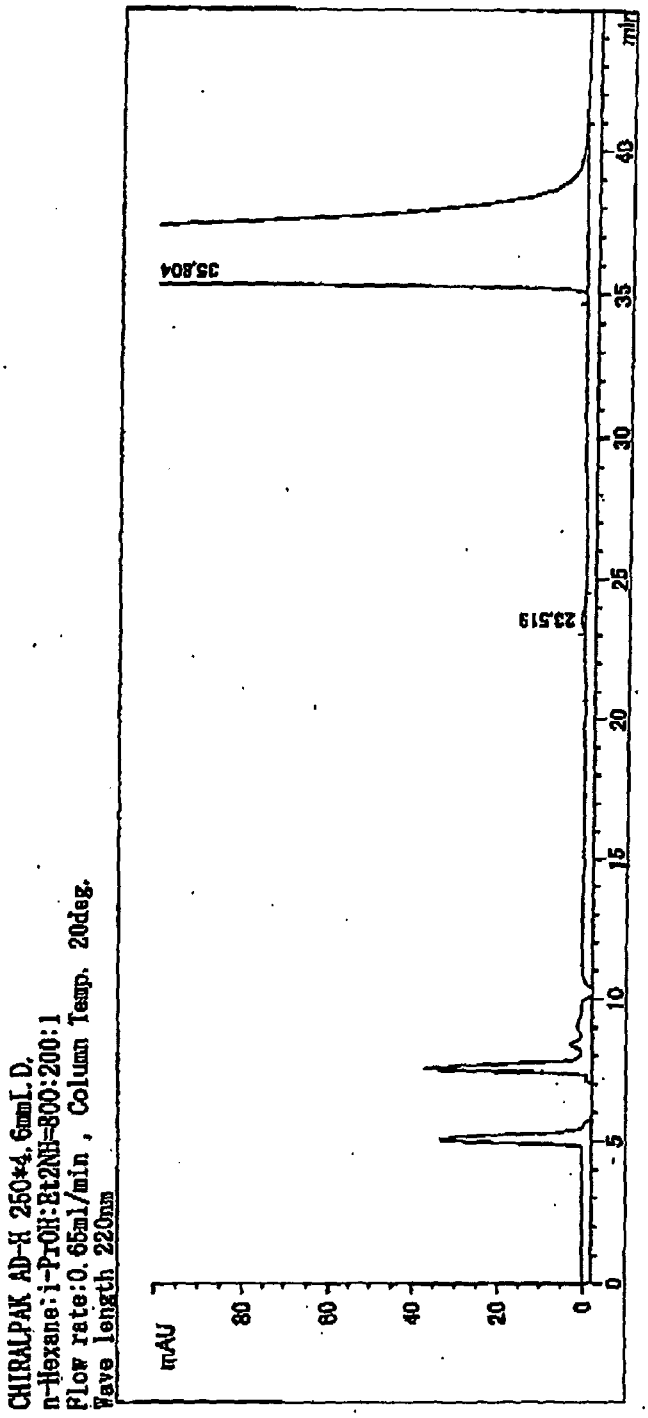
CHIRALPAK AD-H 250*4.6mmI.D.
n-Hexane:i-PrOH:Et2NH=800:200:1
Flow rate:0.65ml/min , Column Temp. 20deg.
Wave length 220nm
mAU
80
60
40
20
0
0
5
10
15
20
25
30
35
40
min
23.519
35.804

**INTERNATIONAL SEARCH REPORT**

International application No.
PCT/JP2005/001747

A. CLASSIFICATION OF SUBJECT MATTER
Int.Cl$^7$ C07D453/02, A61K31/439, A61P13/02, 27/08, 1/12

According to International Patent Classification (IPC) or to both national classification and IPC

B. FIELDS SEARCHED

Minimum documentation searched (classification system followed by classification symbols)
Int.Cl$^7$ C07D453/02, A61K31/439, A61P13/02, 27/08, 1/12

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-104968 A (Yamanouchi Pharmaceutical Co., Ltd.), 10 April, 2002 (10.04.02), Claim 5 (Family: none) | 1-18 |
| X | WO 03/006019 A1 (Yamanouchi Pharmaceutical Co., Ltd.), 23 January, 2003 (23.01.03), Particularly, page 6 & EP 1405638 A1 07 April, 2004 (07.04.04), & US 2004-0138252 A 15 July, 2004 (15.07.04) | 1-18 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 March, 2005 (02.03.05) | 22 March, 2005 (22.03.05) |
| Name and mailing address of the ISA/ Japanese Patent Office | Authorized officer |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 801067 A
- WO 20036019 A
- JP 2002104968 A


### Non-patent literature cited in the description


- *Current Opinion in Central & Peripheral Nervous System Investigational Drugs,* 2000, vol. 2 (3), 321-325
- *Drugs of the Future,* 1999, vol. 24 (8), 871-874
- *Naunyn-Schmiedeberg's Archives of Pharmacology,* 2002, vol. 366 (2), 97-103
- *Japanese Journal of Pharmacology,* 2001, vol. 86 (3), 281-288
- *The Abstract of Lectures at the Memorial Symposium for the Foundation and Establishment of Japan Process Chemistry Association,* 04 July 2002, 85-86